Europäisches Patentamt

European Patent Office

Office européen des brevets

(19)

(11) Publication number: **0 264 914**
A2

(12)

# EUROPEAN PATENT APPLICATION

(21) Application number: 87115350.8

(22) Date of filing: 20.10.87

(51) Int. Cl.4: **C12N 15/00** , C12P 13/22 , C12N 1/20 , //(C12N1/20,C12R1:15,1:13)

The applicant has filed a statement in accordance with Rule 28 (4) EPC (issue of a sample only to an expert). Accession number(s) of the deposit(s): FERM BP-454, FERM BP-864, FERM BP-457, FERM BP-1120, FERM BP-1121, FERM BP-459, FERM BP-1123, FERM BP-1122.

(30) Priority: 21.10.86 JP 250013/86

(43) Date of publication of application:
27.04.88 Bulletin 88/17

(84) Designated Contracting States:
DE FR GB

(71) Applicant: KYOWA HAKKO KOGYO CO., LTD.
6-1, Ohte-Machi Itchome
Chiyoda-ku Tokyo(JP)

(72) Inventor: Katsumata, Ryoichi
CI-Heights H-801 1380, Yamazaki-machi
Machida-shi Tokyo(JP)
Inventor: Ozaki, Akio
2-3-108, Kyowa-cho
Hofu-shi Yamaguchi-ken(JP)
Inventor: Ikeda, Masato
2-5-5, Futaba
Sagamihara-shi Kanagawa-ken(JP)

(74) Representative: Vossius & Partner
Siebertstrasse 4 P.O. Box 86 07 67
D-8000 München 86(DE)

(54) Process for producing L-phenylalanine.

(57) The invention relates to a bacterial process for producing L-phenylalanine. The process utilizes a Corynebacterium or Brevibacterium host which is transformed with a recombinant DNA. The recombinant DNA harbors a DNA containing a gene which encodes 3-deoxy-D-arabino-hepturosonate-7-phosphate synthase, chorismate mutase and prephenate dehydratase. The transformant is then cultured in order to accumulate L-phenylalanine in the culture medium and the L-phenylalanine is recovered therefrom.

EP 0 264 914 A2

# PROCESS FOR PRODUCING L-PHENYLALANINE

In the production of L-phenylalanine by fermentation using a microorganism belonging to the genus Corynebacterium or Brevibacterium, mutant strains carrying an amino acid-requiring mutation and/or an amino acid analogue-resistant mutation have been employed [J. Agric. Chem. Soc., 50 (1). p.R. 79 (1976)].

On the other hand, L-phenylalanine-producing strains have been constructed by recombinant DNA technology. For example, L-phenylalanine-producing strains carrying a recombinant DNA containing a gene coding for 3-deoxy-D-arabino-hepturosonate-7-phosphate synthase (hereinafter referred to as DS), or a gene coding for chorismate mutase (hereinafter referred to as CM) and a gene coding for prephenate dehydratase (hereinafter referred to as PD) (the genes are hereinafter sometimes referred to as DS gene, CM gene and PD gene, respectively), are known [JP-A-24192/85 (EP-A-136359), JP-A-260892/86 and JP-A-124375/86].

With the recent increase in the demand for L-phenylalanine, improved processes for the industrial production thereof are desired. As a result of intensive studies to construct a new strain with higher L-phenylalanine productivity by recombinant DNA technology, the present inventors have found that L-phenylalanine productivity can be improved by introducing, into a microorganism belonging to the genus Corynebacterium or Brevibacterium, a recombinant DNA containing all of genetic information responsible for synthesis of DS, CM and PD, and have established the present invention. As L-phenylalanine-producing strains carrying a recombinant DNA, those carrying a recombinant DNA containing DS gene (JP-A-124375/86), or CM and PD genes [JP-A-24192/85 (EP-A-136359) and JP-A-260892/86] are known, but one carrying a recombinant DNA containing DS, CM and PD genes all together has not been known. The present inventors have first found that the productivity of L-phenylalanine can be considerably increased by amplification of the three genes.

The present invention relates to a process for producing L-phenylalanine which comprises introducing, into a microorganism belonging to the genus Corynebacterium or Brevibacterium, a recombinant DNA containing a DNA fragment that bears all of genetic information responsible for synthesis of DS, CM and PD; culturing the transformant in a culture medium until a recoverable amount of L-phenylalanine is accumulated in the culture broth, and recovering L-phenylalanine therefrom. Thus, the present invention falls within the field of bioindustry, specifically the field of manufacture of L-phenylalanine which is useful in medicines and foods.

Fig. 1 is a restriction enzyme cleavage map of pEaroG-pheA1 and illustrates the steps for constructing the plasmid, where the gene is transcribed in the direction of the arrow.

Fig. 2 and Fig. 3 are restriction enzyme cleavage maps of pCDS-CM1 and pCDCP1, respectively and illustrate the steps for constructing the respective plasmid. The chromosomal DNA fragment indicated by solid box contains the DS gene; that indicated by hatched box, the CM gene; and that indicated by stippled box, the PD gene.

The size of plasmid is expressed in kilobase (kb).

The present invention provides a process for producing L-phenylalanine, which comprises introducing, into a microorganism belonging to the genus Corynebacterium or Brevibacterium, a recombinant DNA containing a DNA fragment that bears all of genetic information responsible for synthesis of DS, CM and PD, culturing the transformant thus obtained in a culture medium until a recoverable amount of L-phenylalanine is accumulated in the culture broth, and recovering L-phenylalanine therefrom. As the DNA fragment to be used in the present invention, those isolated from microorganisms belonging to the genus Escherichia, Corynebacterium or Brevibacterium may be mentioned.

Any microorganism can be used as a source of the respective genes coding for DS, CM and PD, so long as it has a capability of synthesizing phenylalanine. Particularly, DS, CM and PD genes isolated from procaryotes, for example, bacteria belonging to the genus Escherichia, Corynebacterium or Brevibacterium are desirable, and genes isolated from aromatic amino acid-producing mutants derived from these bacteria are the most preferred.

All the glutamic acid-producing coryneform bacteria belonging to the genus Corynebacterium or Brevibacterium can be used as the host microorganism. The following strains are exemplars of the host microorganism:

Corynebacterium glutamicum ATCC 13032
Corynebacterium acetoacidophilum ATCC 13870
Corynebacterium herculis ATCC 13868
Corynebacterium lilium ATCC 15990
Brevibacterium divaricatum ATCC 14020

Brevibacterium flavum ATCC 14067
Brevibacterium immariophilum ATCC 14068
Brevibacterium lactofermentum ATCC 13869
Brevibacterium thiogenitalis ATCC 19240

Above all, L-phenylalanine, L-tryptophan or L-tyrosine-producing strains derived from the above microorganisms by mutation can be preferably used. These mutants can be obtained by selecting strains that carry amino acid requiring-mutation and/or amino acid analogue-resistant mutation [J. Agric. Chem. Soc., 50 (1), p.R. 79 (1976)].

When an L-tryptophan-or L-tyrosine-producing microorganism is used as a host microorganism, the L-tryptophan or L-tyrosine production is converted to the L-phenylalanine production, and thus a strain having an ability to produce a considerable amount of L-phenylalanine can be obtained. It has been found for the first time that a considerable amount of L-phenylalanine can be produced by introducing the recombinant DNA of the present invention into the L-tryptophan-or L-tyrosine-producing microorganism.

The vector to be used for incorporation of the DNA is restricted only to the extent that it is autonomously replicable in microorganisms belonging to the genus Corynebacterium or Brevibacterium. Particularly suitable plasmids are pCG1 (JP-A-134500/82, EP-A-58889, US-A-4617267), pCG2 (JP-A-35197/83, EP-A-73062, US-A-4489160), pCG4 and pCG11 (JP-A-183799/82, EP-A-63763, US-A-4500640), pCE54 and pCB101 (JP-A-105999/83, EP-A-82485), pCE51 (JP-A-34197/85, EP-A-136359, pCE52 and pCE53 [Mol. Gen. Genet., 196 , 175 (1984)].

The recombinant DNA comprising a donor DNA containing DS gene and the vector DNA can be obtained together with a mixture of various recombinants, according to a conventional method, for example, by digesting chromosomal DNA and vector plasmid with a restriction enzyme in vitro, followed by treatment with a DNA ligase or by digesting chromosomal DNA and vector plasmid with a restriction enzyme, followed by treatment of cleaved terminals with terminal transferase, DNA polymerase, etc. and successive treatment with a DNA ligase [Methods in Enzymology, 68 (1979)]. A recombinant DNA containing DS gene can be obtained by transforming a DS-deficient mutant strain belonging to the genus Corynebacterium or Brevibacterium with the said mixture of recombinants according to the method using protoplasts as disclosed in JP-A-186492/82 (EP-A-63764) and JP-A-186489/82 (EP-A-64680), selecting a transformant wherein the deficient character is complemented and isolating a plasmid from the transformant.

· Likewise, a recombinant DNA comprising a donor DNA containing CM gene or PD gene and a vector DNA can be obtained (JP-A-24192/85, EP-A-136359), that is, by transforming a phenylalanine-and tyrosine-requiring (due to deficiency of CM gene) mutant strain or a phenylalanine-requiring (due to deficiency of PD gene) mutant strain of the genus Corynebacterium or Brevibacterium with a mixture of recombinants of the chromosomal DNA and the vector DNA, and by selecting a phenylalanine-and tyrosine-non-requiring transformant or a phenylalanine-non-requiring transformant, followed by isolation of the recombinant DNA containing CM gene or PD gene therefrom.

In place of directly obtaining the desired recombinant DNA from a transformant of the genus Corynebacterium or Brevibacterium, the desired recombinant DNA can also be obtained by using the host-vector system such as Escherichia coli, where the genetic recombination technique has already been established. That is, mutants of Escherichia coli deficient in DS gene, CM gene or PD gene are transformed with in vitro ligation mixtures of a donor DNA and a vector DNA, and the transformant wherein the deficient character is complemented is selected. The cloned DNA isolated from the transformant and a vector DNA autonomously replicable in microorganisms of the genus Corynebacterium or Brevibacterium are digested with a restriction enzyme in vitro and then subjected to ligation reaction with a DNA ligase. Then, a mutant of the genus Corynebacterium or Brevibacterium deficient in DS gene, CM gene or PD gene is transformed with the ligation reaction mixture, and a transformant wherein the deficient character is complemented is selected. Thus, the desired recombinant DNA can also be obtained by this method (JP-A-260892/86).

The DNA fragment containing DS gene, CM gene or PD gene thus obtained can be subjected to further recombination to obtain a recombinant DNA containing all of DS, CM and PD genes. By introducing the recombinant DNA into a host microorganism, the three genes can be amplified. The three genes can also be amplified, if they are contained in different vector plasmids capable of coexisting in the same cell and these plasmids are simultaneously introduced in a host microorganism. In both cases, the increased productivity of L-phenylalanine can be achieved.

When chromosomal DNA of a wild strain of the genus Escherichia, Corynebacterium or Brevibacterium is used as the donor DNA in the steps described above, a recombinant DNA containing all of DS, CM and PD genes of wild-type is introduced into a microorganism belonging to the genus Corynebacterium or Brevibacterium. However, it is known that in microorganisms of the genus Corynebacterium or Brevibacterium, DS and CM are subjected to feedback inhibition by phenylalanine and tyrosine, and that PD is subjected to feedback inhibition by phenylalanine [Agric. Biol. Chem., 39, 351 (1975)]. Thus, it is preferable to use a recombinant DNA containing the genes encoding DS, CM and PD released from such inhibition, in order to ensure higher L-phenylalanine productivity in a microorganism of the genus Corynebacterium or Brevibacterium. The mutant DS, CM and PD genes are cloned by using the chromosomal DNA of a mutant strain of the genus Corynebacterium or Brevibacterium , whose DS, CM or PD is released from feedback inhibition, as a passenger DNA. Cloning can be carried out by complementation of DS, CM or PD deficiency in mutant strains of Corynebacterium or Brevibacterium, or by selecting a transformant resistant to a phenylalanine analogue such as p-fluorophenylalanine (hereinafter referred to as PFP) with the use of the wild-type strains as recipient. A recombinant plasmid containing mutant DS, CM and PD genes can be prepared in the same manner as in construction of the recombinant plasmid containing the wild-type genes. Alternatively, a similar recombinant DNA containing mutant DS, CM and PD genes can also be obtained by in vitro mutagenesis of a recombinant plasmid containing wild-type genes according to the method described in Mol. Gen. Genet. 145, 101 (1978), or by in vivo mutagenesis of a strain carrying the recombinant plasmid.

The recombinant plasmid containing wild-type or mutant DS, CM and PD genes can be introduced into a microorganism of the genus Corynebacterium or Brevibacterium according to the aforementioned transformation method using a protoplast.

Production of L-phenylalanine by strains carrying these recombinant plasmids can be carried out in a conventional manner used for the production of amino acids by fermentation. That is, the transformant is cultured in an ordinary medium containing carbon sources, nitrogen sources, inorganic compounds, amino acids, vitamins, etc. under aerobic conditions, and thus, L-phenylalanine can be formed and accumulated in the culture broth. Then, L-phenylalanine is recovered from the culture broth.

As the carbon source, carbohydrates such as glucose, glycerol, fructose, sucrose, maltose, mannose, starch, starch hydrolyzate, molasses, etc.; polyalcohols; and various organic acids such as pyruvic acid, fumaric acid, lactic acid, acetic acid, etc. can be used. Furthermore, hydrocarbons, alcohols, etc. can be used, depending upon the assimilability of the microorganism to be used. Cane molasses is preferably used.

As the nitrogen source, ammonia; various inorganic and organic ammonium salts such as ammonium chloride, ammonium sulfate, ammonium carbonate, ammonium acetate, etc., urea and other nitrogen-containing compounds as well as nitrogen-containing organic compounds such as peptone, NZ-amine, meat extract, yeast extract, corn steep liquor, casein hydrolyzate, fish meal or its digested product, chrysalis hydrolyzate, etc. can be used.

As the inorganic compound, dipotassium hydrogenphosphate, potassium dihydrogenphosphate, ammonium sulfate, ammonium chloride, magnesium sulfate, sodium chloride, ferrous sulfate, manganese sulfate. calcium carbonate, etc. can be used. If vitamins, amino acids, etc. required for the growth of the microorganism are supplied by other medium components described above, it is not necessary to add these specific nutrients separately to the medium.

Culturing is carried out under aerobic conditions by shaking culture, aeration-stirring culture, etc. The preferred culturing temperature is generally 20-40°C. It is desirable that the pH of the culture medium is maintained around the neutrality. After culturing for 1-5 days under these conditions, a recoverable amount of L-phenylalanine is accumulated in the culture broth. After completion of the culturing, the cells are removed from the culture broth, and L-phenylalanine is recovered from the supernatant by a known method. for example, by activated carbon treatment, ion exchange resin treatment, etc.

Thus, L-phenylalanine can be produced in high yield by using a strain of the genus Corynebacterium or Brevibacterium carrying a recombinant DNA containing all of DS, CM and PD genes.

In the present specification, a case where the productivity of L-phenylalanine is increased by introducing a recombinant DNA containing all of DS, CM and PD genes into Corynebacterium glutamicum is given. and the intended object can be also attained with any other glutamic acid-producing coryneform bacteria in place of Corynebacterium glutamicum.

In spite of many common microbiological properties, microorganisms with high glutamic acid productivity (so-called glutamic acid-producing microorganisms) are classified to various species by researchers and even genera such as Corynebacterium and Brevibacterium probably because of their industrial importance. However, it has been pointed out that these microorganisms should be classified as one species because they have homology in the amino acids in the cell walls and the base composition of DNA. Further, it has been reported that these microorganisms have more than 70-80% homology in DNA-DNA hybridization, indicating that the microorganisms are very closely related [refer to Komatsu, Y.: Report of the Fermentative Research Institute, No. 55, 1 (1980), and Suzuki, K., Kaneko, T., and Komagata, K.: Int. J. Syst. Bacteriol.,

31, 131 (1981)]. Considering the above-mentioned very close relationship of glutamic acid-producing microorganisms, it is readily assumed that the present invention is applicable to all of the glutamic acid-producing microorganisms. The effect of the present invention depends on whether the recombinant DNA autonomously replicates in the glutamic acid-producing microorganism and whether DS, CM and PD genes are expressed, and so slight difference of such DNA homology between glutamic acid-producing coryneform bacteria are negligible. That the glutamic acid-producing microorganisms have the common function to allow replication of plasmids and expression of genes is apparent from the fact that plasmid pCG4 which is isolated from Corynebacterium glutamicum 225-250 (JP-A-183799/82, EP-A-63763, US-A-4500640) and which has spectinomycin and/or streptomycin resistance gene(s) can be generally replicated and that the gene(s) can be expressed in glutamic acid-producing coryneform bacteria such as strains of the genera Corynebacterium and Brevibacterium (JP-A-186492/82, EP-A-63764).

The species to which the process for constructing an L-phenylalanine-producing microorganism by introducing a recombinant DNA containing all of DS, CM and PD genes of the present invention is applicable, include not only Corynebacteriumglutamicum, but also all of glutamic acid-producing coryneform bacteria including the bacteria of the genera Corynebacterium and Brevibacterium.

Certain specific embodiments of the present invention are illustrated by the following representative examples and reference example.

Example 1

Production of phenylalanine by a strain carrying a recombinant plasmid containing all of DS, CM and PD genes of Escherichia coli K-12:

(1) Preparation of chromosomal DNA of Escherichia coli JA194 strain, plasmid pPHEA1 and vectors pBR322 and pCE51:

Chromosomal DNA of Escherichia coli JA194, derived from Escherichia coli K-12 [Proc. Natl. Acad. Sci., 94 487-491 (1977)] was prepared in the following manner:

At first, 20 mℓ of a seed culture was inoculated in 400 mℓ of L-broth medium (medium containing 10 g of Bactotryptone, 5 g of Bacto-yeast extract and 5 g of NaCl in 1 ℓ of water, adjusted to pH 7.0, hereinafter referred to as LB medium), cultured by shaking at 37°C. The cells grown to late logarithmic phase were collected from the culture broth, and high molecular chromosomal DNA was isolated from the collected cells according to the method of Saito-Miura [Biochem. Biophys. Acta. 72, 619 (1963)].

Plasmid pPHEA1 is a plasmid which was constructed by inserting an EcoRI-BamHI-cleaved DNA fragment containing pheA gene coding for CM and PD of Escherichia coli into plasmid pBR322 of Escherichia coli at the EcoRI-BamHI-cleavage site as described in Japanese Published Unexamined Patent Application No. 260892/86 (the method of preparation thereof is shown in Reference Example).

pCE51 used as a vector (JP-A-34197/85, EP-A-136359) is a plasmid constructed by combining plasmid pCG1 autonomously replicable in Corynebacterium glutamicum (JP-A-134500/82, EP-A-58889, US-A-4617267) with plasmid pGA22 autonomously replicable in Escherichia coli (JP-A-134500/82, EP-A-58889, US-A-4617267) with plasmid pGA22 autonomously replicable in Escherichia coli [J. Bacteriol., 140, 400 (1979)].

pPHEA1, pBR322 and pCE51 were isolated from cultured cells of Escherichia coli JA194 strains containing each of the plasmids in the following manner.

At first, 20 mℓ of a seed culture of a strain carrying pPHEA1 or pBR322 and 20 mℓ of a seed culture of a strain carrying pCE51 were inoculated into 400 mℓ of LB medium containing 100 μg/mℓ ampicillin and 400 mℓ of LB medium containing 20 μg/mℓ kanamycin, respectively, and cultured until the absorbance (OD) at 660 nm reached about 0.7 (the absorbance was measured at 660 nm unless otherwise specified). The cultured cells were collected, washed with a TES buffer solution (pH 8.0) comprising 0.03 M Tris-(hydroxymethyl)aminomethane (hereinafter referred to as Tris), 0.005 M disodium ethylenediamine tetraacetate (hereinafter referred to as EDTA) and 0.05 M NaCl, then suspended in 10 mℓ of a lysozyme solution (pH 8.0) comprising 25% sucrose, 0.05 M Tris, 0.1 M NaCl and 0.8 mg/mℓ lysozyme and subjected to reaction at 37°C for one hour. Then, 2.4 mℓ of 5 M NaCl, 0.6 mℓ of 0.5 M EDTA (pH 8.5) and 4.4 mℓ of a solution composed of 4% sodium laurylsulfate and 0.7 M NaCl were successively added to the reaction solution, and the mixture was gently stirred and placed on ice for 15 hours. The mixture was transferred into a centrifuge tube and centrifuged at 69,400 × g at 4°C for 60 minutes to recover the supernatant. Then, polyethylene glycol (PEG) 6,000 (a product of Nakarai Chemicals Co., Ltd.) in an amount corresponding to 10% by weight was added thereto and the mixture was gently stirred, and then placed on ice. After 10 hours, the mixture was centrifuged at 1,500 $^\times$ g for 10 minutes to recover pellets. Then, 5 m$\ell$ of TES buffer solution was added to gradually dissolve the pellets. Then, 2.0 m$\ell$ of 1.5 mg/m$\ell$ ethidium bromide was added to the solution. Cesium chloride was added to adjust the density of the solution to 1.580. Then, the solution was subjected to ultra-centrifugation at 105,000 $^\times$ g at 18°C for 48 hours, and a high density band at the lower position of the centrifuge tube, detected under ultraviolet irradiation, was withdrawn from the side of the centrifuge tube through a syringe, to recover pPHEA1, pBR322 or pCE51 plasmid DNA. The fraction was treated 5 times with an equal volume of isopropyl alcohol solution [90% (V/V) of isopropyl alcohol and 10% (V/V) TES buffer solution (containing a saturated amount of cesium chloride)] to remove ethidium bromide by extraction. Then, the solution was dialyzed against TES buffer solution.

(2) Cloning of aroG gene:

Five units of restriction enzyme HindIII (product of Takara Shuzo Co., Ltd.) was added to 100 $\mu\ell$ of reaction buffer solution for restriction enzyme HindIII (comprising 10 mM Tris, 6 mM MgCl$_2$ and 50 mM NaCl, pH 7.5) containing 3 $\mu$g of pBR322 plasmid DNA prepared in step (1). Separately, 5 units of HindIII was added to 100 $\mu\ell$ of reaction buffer solution for restriction enzyme HindIII containing 3 $\mu$g of chromosomal DNA of Escherichia coli JA194 strain prepared in step (1). The respective mixtures were subjected to reaction at 37°C for 60 minutes. To each of the two reaction solutions were added 5 $\mu\ell$ of 2 M NaCl and 5 units of restriction enzyme SalI (product of Takara Shuzo Co., Ltd.) and the reaction solutions were further subjected to reaction at 37°C for 60 minutes. Then, the reaction was stopped by heating the reaction solutions at 65°C for 10 minutes. Both reaction solutions were mixed, and then to the mixture were added 40 $\mu\ell$ of a buffer solution for T4 ligase (comprising 660 mM Tris, 66 mM MgCl$_2$ and 100 mM dithiothreitol, pH 7.5), 40 $\mu\ell$ of 5 mM ATP, 1 $\mu\ell$ of T4 ligase (1 unit/$\mu\ell$, product of Takara Shuzo Co., Ltd.) and 110 $\mu\ell$ of water, and the mixture was subjected to reaction at 12°C for 16 hours.

The ligase reaction mixture was used for transformation with Escherichia coli AB3248 strain (FERM BP-865) deficient in gene coding for DS (aroF363, aroG365, aroH367, thi, his, pro, arg, ilv, T6$^r$, F$^-$) [J. Bacteriol. 93, 237 (1967)] as a recipient. An overnight seed culture broth of Escherichia coli AB3248 strain was inoculated in LB medium to prepare competent cells according to the method of M. Dagert, et al. [Gene, 6, 23 (1979)].

Then, 50 $\mu\ell$ of the ligase reaction mixture was added to 0.2 m$\ell$ of a suspension containing competent cells at a concentration of about 10$^9$ cells/m$\ell$ and the mixture was left standing on ice for 10 minutes. Then, the mixture was heated at 37°C for 5 minutes and admixed with 2 m$\ell$ of LB medium. Then, the mixture was left standing at 37°C for 90-120 minutes. Then, the cells were centrifugally washed twice with physiological saline solution and spread onto M9 plate medium (medium comprising 2 g of glucose, 1 g of NH$_4$Cl, 6 g of Na$_2$HPO$_4$, 3 g of KH$_2$PO$_4$, 0.1 g of MgSO$_4$•7H$_2$O, 15 mg of CaCl$_2$•2H$_2$O, 4 mg of thiamine hydrochloride and 15 g of agar in 1 $\ell$ of water, adjusted to pH 7.2), containing 50 u$\ell$/m$\ell$ each of histidine, proline, arginine, isoleucine and valine. Colonies grown on M9 plate medium were spread onto LB plate medium (LB medium containing 1.6% agar, pH 7.0) containing 100 $\mu$g/m$\ell$ ampicillin and LB plate medium containing 20 $\mu$g/m$\ell$ tetracycline, and transformants grown on the ampicillin-containing medium and failing to grow on the tetracycline-containing medium were selected.

From the thus selected ampicillin-resistant and tetracycline-sensitive transformants of growing on M9 plate medium containing histidine, proline, arginine, isoleucine and valine, plasmid DNAs were isolated in the same manner as in step (1). Plasmid pAROG1 isolated from one of the transformants was digested with various restriction enzymes, and analyzed by agarose gel electrophoresis. It was found that the plasmid pAROG1 has a structure wherein HindIII-SalI-cleaved DNA fragment of 6 kilobases (hereinafter referred to as kb) is inserted in a region between HindIII-and Sal I-cleavage sites of pBR322.

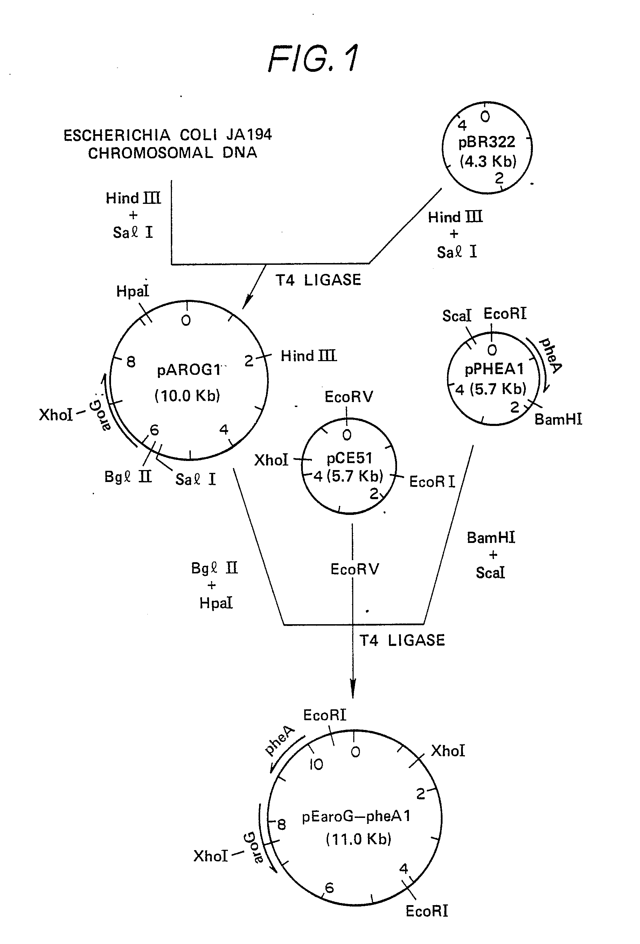

Furthermore, the Hin dIII-SalI-cleaved DNA fragment had the same restriction enzyme cleavage sites as those of aroG gene, as reported by W.D. Davies, et al. [Nucl. Acids Res., 10, 4045 (1982)] (see Fig. 1), and the growth of Escherichia coli AB3248 strain carrying pAROG1 was completely inhibited on M9 plate medium containing 500 $\mu$g/m$\ell$ phenylalanine. Thus, it was found from these facts that aroG gene of Escherichia coli is encoded on the DNA fragment of about 6 kb inserted in pAROG1.

(3) Construction of recombinant plasmid containing aroG and pheA genes together:

A DNA fragment containing aroG gene isolated from pAROG1, and a DNA fragment containing pheA gene isolated from pPHEA1, are combined with pCE51.

Five units each of restriction enzymes BglII and HpaI (products of Takara Shuzo Co., Ltd.) were added to 100 μl of a reaction solution (comprising 10 mM Tris, 6 mM MgCl₂ and 100 mM NaCl, pH 7.5) containing 3 μg of pAROG1 plasmid DNA, and 5 units each of restriction enzymes ScaI and Bam HI (products of Takara Shuzo Co., Ltd.) were added to 100 μl of a reaction solution (comprising 10 mM Tris, 6 mM MgCl₂ and 100 mM NaCl, pH 7.5) containing 3 μg of pPHEA1 plasmid DNA, and both mixtures were subjected to reaction at 37°C for 60 minutes.

Separately, 5 units of restriction enzyme EcoRV (product of Takara Shuzo Co., Ltd.) was added to 100 μl of a reaction solution (comprising 10 mM Tris, 6 mM MgCl₂ and 100 mM NaCl, pH 7.5) containing 3 μg of pCE51 plasmid DNA, and the mixture was subjected to reaction at 37°C for 60 minutes. Reaction was stopped by heating the respective mixtures at 65°C for 10 minutes. Then, the three reaction mixtures were mixed, 40 μl of a buffer solution for T4 ligase, 40 μl of 5 mM ATP, 1 μl of T4 ligase (1 unit/μl, product of Takara Shuzo Co., Ltd.) and 20 μl of water were added thereto, and then the mixture was subjected to reaction at 12°C for 16 hours.

Escherichia coli AB3248 strain was transformed with this ligase reaction mixture in the same manner as in step (2), and the cells were centrifugally washed twice with physiological saline solution, and then spread onto M9 plate medium containing 50 μg/ml each of histidine, proline, arginine, isoleucine and valine. Colonies grown on M9 plate medium were collected by scraping, and plasmid DNA was isolated in the same manner as in step (1).

Escherichia coli GS245 strain (pheA905, araD139, ΔlacU169, ΔglyA, strA, thi) [Gene, 14, 63 (1981)] was transformed with the isolated plasmid DNA in the same manner as in step (2). From colonies grown on M9 plate medium containing 50 μg/ml glycine, were selected transformants grown on LB plate medium containing 20 μg/ml kanamycin.

From the thus obtained transformants, plasmid DNA was isolated in the same manner as in step (1). Plasmid pEaroG-pheA1 obtained from one of the transformants was digested with various restriction enzymes and analyzed by agarose gel electrophoresis, and it was found that the plasmid pEaroG-pheA1 has a structure wherein a Bgl II-HpaI-cleaved DNA fragment containing aroG gene of 2.8 kb and a ScaI-BamHI-cleaved DNA fragment containing pheA gene of 2.5 kb are inserted in pCE51 at the EcoRV-cleavage site (see Fig. 1).

(4) Preparation of mutant plasmid pEaroG-pheA2 in which DS encoded by aroG gene is released from inhibition by phenylalanine:

Escherichia coli AB3248 strain carrying pEaroG-pheA1 obtained in step (3) was cultured in LB medium containing 20 μg/ml kanamycin. Then, the cells grown to late logarithmic phase were centrifugally washed twice with 50 mM Tris-maleate buffer solution (comprising 20 mM Tris and 50 mM maleic acid, pH 6.0) and treated with 50 mM Tris-maleate buffer solution (pH 6.0) containing 200 μg/ml N-methyl-N′-nitro-N-nitrosoguanidine at room temperature for 30 minutes. The treated cells were centrifugally washed twice with 50 mM Tris-maleate buffer solution (pH 6.0), and then the washed cells were cultured in LB medium containing 20 μg/ml kanamycin at 30°C for 16 hours. Plasmid DNA was isolated therefrom in the same manner as in step (1).

Escherichia coli AB3248 strain was transformed with the isolated plasmid in the same manner as in step (2). Selection of transformants was carried out on M9 plate medium containing 6 mg/ml phenylalanine and 50 μg/ml each of histidine, proline, arginine, isoleucine and valine. From the colonies, transformants capable of growing on M9 plate medium containing 6 mg/ml phenylalanine and 50 μg/ml each of histidine, proline, arginine, isoleucine and valine and on LB plate medium containing 20 μg/ml kanamycin were selected.

Plasmids isolated from the thus obtained transformants could confer a phenylalanine-resistance on Escherichia coli AB3248 strain. A plasmid isolated from one of the transformants was named pEaroG-pheA2.

(5) Transformation of Corynebacterium glutamicum with pEaroG-pheA2 and preparation of mutant plasmid pEaroG-pheA3 in which CM and PD encoded by the pheA gene are released from inhibition by phenylalanine:

At first, 4 mℓ of a seed culture of Corynebacterium glutamicum ATCC 39019 (strain carrying a lysozyme-sensitive mutation derived from Corynebacterium glutamicum ATCC 31833) was inoculated in 40 mℓ of NB medium (medium containing 20 g of bouillon powder and 5 g of yeast extract in 1 ℓ of water, adjusted to 7.2) and cultured by shaking at 30°C. The cells were collected when OD reached 0.6, and suspended to a concentration of about $10^9$ cells/mℓ in 10 mℓ of RCGP medium [medium containing 5 g of glucose, 5 g of casamino acid, 2.5 g of yeast extract, 3.5 g of $K_2HPO_4$, 1.5 g of $KH_2PO_4$, 0.41 g of $MgCl_2 \cdot 6H_2O$, 10 mg of $FeSO_4 \cdot 7H_2O$, 2 mg of $MnSO_4 \cdot 4-6H_2O$, 0.9 mg of $ZnSO_4 \cdot 7H_2O$, 0.04 mg of $(NH_4)_6Mo_7O_{24} \cdot 4H_2O$, 30 μg of biotin, 2 mg of thiamine hydrochloride, 135 g of disodium succinate and 30 g of polyvinyl pyrrolidone having a molecular weight of 10.000, in 1 ℓ of water, pH 7.6] containing 1 mg/mℓ lysozyme. The suspension was transferred into an L-type test tube and gently shaken at 30°C for 5 hours to prepare protoplasts.

Then, 0.5 mℓ of the protoplast suspension was taken in a small test tube and centrifuged at 2.500 × g for 5 minutes. Then the protoplasts were suspended in 1 mℓ of TSMC buffer solution (comprising 10 mM $MgCl_2$, 30 mM $CaCl_2$, 50 mM Tris and 400 mM sucrose, pH 7.5) and centrifugally washed. Then, the protoplasts were resuspended in 0.1 mℓ of TSMC buffer solution. Then, 100 μℓ of a 1:1 mixture of TSMC buffer solution at a two-fold concentration and the said pEaroG-pheA2 DNA solution was added to the protoplast suspension, and then the resulting mixture was further admixed with 0.8 mℓ of TSMC buffer solution containing 20% PEG 6.000. After three minutes, 2 mℓ of RCGP medium (pH 7.2) was added thereto, and the mixture was centrifuged at 2.500 × g for 5 minutes to remove a supernatant. The precipitated protoplasts were suspended in 1 mℓ of RCGP medium and 0.2 mℓ of the suspension was spread onto RCGP agar medium (RCGP medium containing 1.4% agar, pH 7.2) containing 200 μg/mℓ kanamycin and cultured at 30°C for 7 days.

Plasmid DNA was isolated from the cultured cells of kanamycin-resistant transformants in the same manner as in step (1). The plasmid obtained from one of the transformants was found to be the same plasmid as pEaroG-pheA2 by the digestion analysis with various restriction enzymes.

Then, Corynebacterium glutamicum ATCC 39019 carrying pEaroG-pheA2 was cultured in NB medium containing 10 μg/mℓ kanamycin. The cells grown to late logarithmic phase were subjected to the mutagenic treatment in the same manner as in step (4), and plasmid DNA was isolated from the cells in the same manner as in step (1).

Corynebacterium glutamicum ATCC 39019 was transformed with the isolated plasmid in the same manner as described above, and kanamycin-resistant colonies grown on RCGP agar medium containing 200 μg/mℓ kanamycin were collected by scraping, centrifugally washed twice with physiological saline solution and then suspended in 1 mℓ of physiological saline solution. The cell suspension was spread on a minimal agar medium MI [medium containing 10 g of glucose, 1 g of $(NH_4)H_2PO_4$, 0.2 g of KCl, 0.2 g of $MgSO_4 \cdot 7H_2O$, 10 mg of $FeSO_4 \cdot 7H_2O$, 0.2 mg of $MnSO_4 \cdot 4-6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 50 μg of biotin, 2.5 mg of p-aminobenzoic acid, 1 mg of thiamine hydrochloride and 16 g of agar in 1 ℓ of water and adjusted to pH 7.2] containing 2000 μg/mℓ PFP and 10 μg/mℓ kanamycin and cultured at 30°C for 5 days to select PFP- and kanamycin-resistant transformants. A plasmid isolated from one of the strains was named pEaroG-pheA3.

DS, CM and PD activities of Corynebacterium glutamicum ATCC 39019 and transformants containing pEaroG-pheA3 were determined. Determination of DS activity was done according to the method of P.R. Sprinavasan, D.B. Sorinson, et al. [J. Biol. Chem., 234, 716 (1959)] and determination of CM and PD activities was done according to the method of R.G.H. Cotton and F. Gibson [Biochem. Biophys. Acta, 100, 76 (1965)].

Activities of DS, CM and PD of the transformant carrying pEaroG-pheA3 shown in Table 1 are expressed relative to those of Corynebacterium glutamicum ATCC 39019.

Furthermore, DS, CM and PD activities of the transformants carrying pEaroG-pheA1, pEaroG-pheA2 or pEaroG-pheA3, were determined when 100 mM phenylalanine was added to the reaction solutions. Their activities shown in Table 2 are expressed as percentages of those without addition of phenylalanine.

Table 1

| Strains | DS | CM | PD |
|---|---|---|---|
| Corynebacterium glutamicum ATCC 39019 | 1.0 | 1.0 | 1.0 |
| Corynebacterium glutamicum ATCC 39019/pEaroG-pheA3 | 16.0 | 8.9 | 8.2 |

Table 2

| Strains | DS | CM | PD |
|---|---|---|---|
| Corynebacterium glutamicum ATCC 39019/pEaroG-pheA1 | 12 | 61 | 5 |
| Corynebacterium glutamicum ATCC 39019/pEaroG-pheA2 | 74 | 66 | 7 |
| Corynebacterium glutamicum ATCC 39019/pEaroG-pheA3 | 74 | 89 | 81 |

(6) Production of phenylalanine by strains carrying pEaroG-pheA3:

Phenylalanine-producing strain Corynebacterium glutamicum K38 (FERM BP-454) [strain having PFP- and paraaminophenylalanine-resistance], tryptophane-producing strain Corynebacterium glutamicum K-55 (FERM BP-864) [strain having phenylalanine-and tyrosine-requirement, and 5-methyltryptophan-, tryptophan hydroxamate-, 6-fluorotryptophan-, 4-methyltryptophan-, PFP-, p-aminophenylalanine-, tyrosine hydroxamate-and phenylalanine hydroxamate-resistance] and tyrosine-producing strain. Corynebacterium glutamicum K43 (FERM BP-457) [strain having 3-aminotyrosine-, p-aminophenylalanine-, PFP-and tyrosine hydroxamate-resistance] were transformed with pEaroG-pheA3.

A protoplast prepared in the following manner was used for the transformation. At first. 4 mℓ of each of seed cultures of K38. K-55 and K43 strains was inoculated in 40 mℓ of semi-synthetic medium SSM [medium containing 20 g of glucose. 10 g of $(NH_4)_2SO_4$. 3 g of urea. 1 g of yeast extract. 1 g of $KH_2PO_4$. 0.4 g of $MgCl_2 \cdot 6H_2O$. 10 mg of $FeSO_4 \cdot 7H_2O$, 0.2 mg of $MnSO_4 \cdot 4-6H_2O$. 0.9 mg of $ZnSO_4 \cdot 7H_2O$, 0.4 mg of $CuSO_4 \cdot 5H_2O$. 0.09 mg of $Na_2B_4O_7 \cdot 10H_2O$. 0.04 mg of $(NH_4)_6Mo_7O_{24} \cdot 4H_2O$. 30 μg of biotin and 1 mg of thiamine hydrochloride in 1 ℓ of water and adjusted to pH 7.2, hereinafter referred to as SSM medium] containing 100 μg/mℓ phenylalanine and tyrosine, respectively, and cultured by shaking at 30°C. When OD reached 0.2, penicillin G was added to a concentration of 0.5 unit/mℓ. Then. the culturing was continued. and when OD reached 0.6, the cultured cells were treated with lysozyme in the same manner as in step (5) to prepare protoplasts. The thus obtained protoplasts were transformed with pEaroG-pheA3 in the same manner as in step (5) to obtain kanamycin-resistant transformants. The respective transformants were cultured in 40 mℓ of SSM medium, and treated with penicillin G in the same manner as above. From the collected. cultured cells. plasmid DNA was isolated in the same manner as in step (1). The plasmid was found to be the same plasmid as pEaroG-pheA3 by digestion analysis with various restriction enzymes.

9

The thus obtained transformants carrying pEaroG-pheA3 were deposited with Fermentation Research Institute (FRI), Agency of Industrial Science and Technology, Japan as Corynebacterium glutamicum K-66 (FERM BP-1120) and Corynebacterium glutamicum K-67 (FERM BP-1121), respectively on August 7, 1986.

Phenylalanine, tyrosine and tryptophan production tests of the transformants and the respective parent strains were carried out in the following manner:

At first, 0.5 mℓ of a seed culture obtained by shaking culture in NB medium at 30°C for 16 hours was inoculated in a test tube containing 5 mℓ of production medium [medium containing 60 g of glucose, 1 g of KH2PO4, 1 g of K2HPO4, 1 g of MgSO4•7H2O, 20 g of (NH4)2SO4, 5 g of corn steep liquor, 10 mg of MnSO4, 30 μg of biotin and 20 g of CaCO3 in 1 ℓ of water and adjusted to pH 7.2] and cultured by shaking at 30°C for 72 hours. After culturing, 1 mℓ of culture broth was admixed with 50 μℓ of 6 N NaOH, and heated at 65°C for 5 minutes to completely dissolve the deposited tyrosine. The culture filtrate was subjected to paper chromatography and after color formation with ninhydrin, yields of L-phenylalanine, L-tyrosine and L-tryptophan were determined by colorimetric quantitative determination. The results are shown in Table 3.

## Table 3

| Strains | L-phenylalanine (g/ℓ) | L-tyrosine (g/ℓ) | L-tryptophan (g/ℓ) |
|---|---|---|---|
| Corynebacterium glutamicum K38 | 5.7 | 0 | 0 |
| Corynebacterium glutamicum K-66 (FERM BP-1120) | 10.2 | 0 | 0 |
| Corynebacterium glutamicum K-55 | 0 | 0 | 4.7 |
| Corynebacterium glutamicum K-67 (FERM BP-1121) | 5.0 | 1.1 | 0.8 |
| Corynebacterium glutamicum K43 | 0 | 5.2 | 0 |
| Corynebacterium glutamicum K43/pEaroG-pheA3 | 6.4 | 1.9 | 0 |

Example 2

Production of phenylalanine by strains carrying a recombinant plasmid containing all of DS, CM and PD genes of Corynebacterium glutamicum K38:

(1) Preparation of chromosomal DNA of Corynebacterium glutamicum K38 strain, plasmid pCS-CM2 and vectors pCE53 and pCG115:

At first, 20 mℓ of a seed culture of Corynebacterium glutamicum K38 (FERM BP-454) cultured in NB medium was inoculated in 400 mℓ of SSM medium, and cultured by shaking at 30°C. When OD reached 0.2, penicillin G was added to the culture medium to a concentration of 0.5 unit/mℓ. The culturing was further continued until OD reached 0.6.

The cells were collected from the culture broth, washed with TES buffer solution, suspended in 10 mℓ of the lysozyme solution and subjected to reaction at 37°C for 4 hours. From the collected cells, high molecular chromosomal DNA was isolated in the same manner as in Example 1, step (1).

Plasmid pCS-CM2 is a plasmid that is constructed by inserting into the Bgl II-cleavage site on plasmid pCG11 autonomously replicable in Corynebacterium glutamicum, the BamHI-cleaved DNA fragment which is isolated from Corynebacterium glutamicum K38 and complements the deficient characters of Corynebacterium glutamicum KY9456 having phenylalanine-and tyrosine-requirement [Agric. Biol. Chem., 39, 331 (1975)], as disclosed in JP-A-24192/85.

pCE53 to be used as a vector is a plasmid obtained by combining plasmid pCG1 of Corynebacterium glutamicum with plasmid pGA22 of Escherichia coli [J. Bacteriol., 140, 400 (1979)]. More specifically, the BglIII-cleaved DNA fragment of pCG1 was inserted into the BamHI-cleavage site outside the tetracycline resistance gene of pGA22 having two BamHI-cleavage sites by the same cohesive ends of both the restriction enzymes. pCE53 has selection markers such as kanamycin resistance gene derived from pGA22, and has one cleavage site for restriction enzyme SalI.

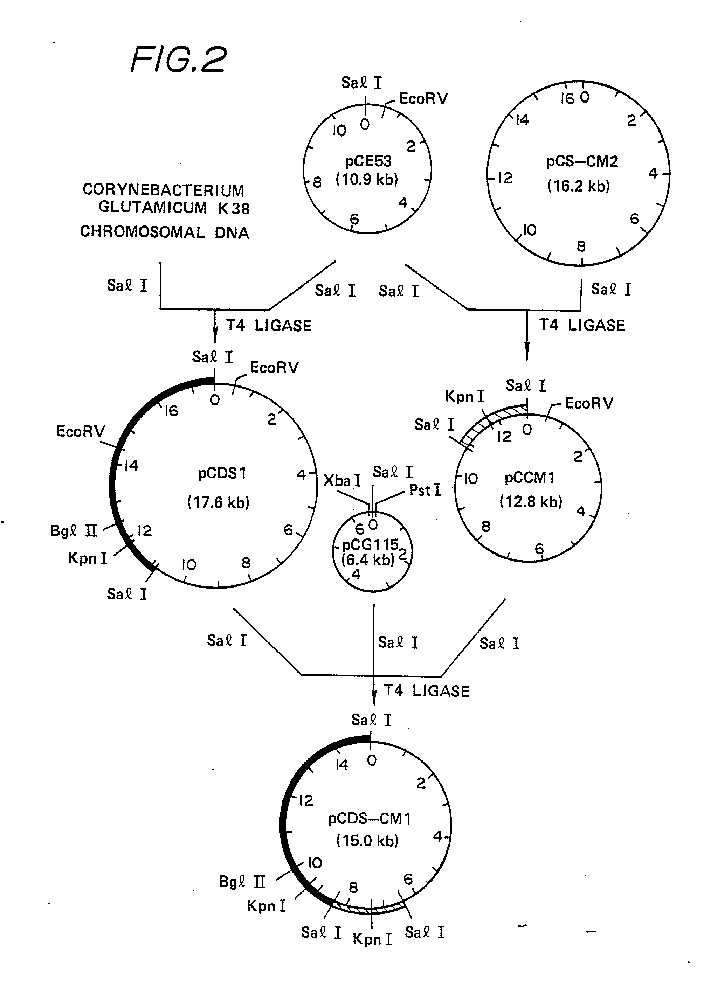

Likewise, pCG115 to be used as a vector is a plasmid obtained by inserting a linker obtained by digesting M13 mp 18RF DNA (product of Takara Shuzo Co., Ltd.) with the restriction enzymes BamHI and PstI, into the BglII-and PstI-cleavage sites of pCG11 by the same cohesive ends. pCG115 is a plasmid having a molecular size of 6.4 kb and has XbaI-, SalI-and PstI-cleavage sites as the single restriction enzyme cleavage site, and confers a phenotype of streptomycin-and/or spectinomycin-resistance on a host (see Fig. 2).

pCS-CM2 was isolated from the cultured cells of Corynebacterium glutamicum K39 (FERM BP-459), and pCE53 and pCG115 were isolated from the cultured cells Corynebacterium glutamicum ATCC 39019 (strain carrying a lysozyme-sensitive mutation derived from ATCC 31833) carrying pCE53 or pCG115 in the following manner:

Corynebacterium glutamicum K39 was cultured by shaking in 400 mℓ of SSM medium at 30°C, treated with penicillin G in the same manner as above, and cultured until OD reached 0.6. On the other hand, Corynebacterium glutamicum ATCC 39019 carrying pCE53 or pCG115 was cultured by shaking in 400 mℓ of NB medium at 30°C until OD reached about 0.7. The cells were collected, and the plasmids were each isolated from the cells in the same manner as in Example 1, step (1).

(2) Cloning of DNA fragment containing DS gene:

At first, 6 units of SalI (product of Takara Shuzo Co., Ltd.) was added to 60 μℓ of a reaction solution for restriction enzyme SalI (comprising 10 mM Tris, 6 mM MgCl₂ and 150 mM NaCl, pH 7.5) containing 3 μg of pCE53 plasmid DNA prepared as above, and the mixture was subjected to reaction at 37°C for 60 minutes. The reaction was stopped by heating the mixture at 65°C for 10 minutes. On the other hand, 6 units ofSalI was added to 140 μℓ of a reaction solution for SalI containing 3 μg of chromosomal DNA of Corynebacterium glutamicum K38 strain, and the mixture was subjected to reaction at 37°C for 60 minutes. The reaction was stopped by heating the mixture at 65°C for 10 minutes.

Then, these two mixtures were mixed and 40 μℓ of a buffer solution for T4 ligase at a 10-fold concentration, 40 μℓ of 5 mM ATP, 0.3 μℓ of T4 ligase (1 unit/mℓ, product of Takara Shuzo Co., Ltd.) and 120 μℓ of water were added thereto, and the mixture was subjected to reaction at 12°C for 16 hours.

The ligase reaction mixture was used to transformation of Corynebacterium glutamicum ATCC 39019. That is, 4 mℓ of a seed culture of Corynebacterium glutamicum ATCC 39019 was inoculated in 40 mℓ of NB medium and cultured by shaking at 30°C. When OD reached 0.6, the cells were collected and suspended in 10 mℓ of RCGP medium containing 1 mg/mℓ lysozyme (pH 7.6) to a concentration of about 10⁹ cells/mℓ, and the suspension was transferred into an L-type test tube and gently shaken at 30°C for 5 hours to prepare protoplasts.

Then, 0.5 mℓ of the protoplast suspension was transferred into a small test tube, centrifuged at 2,500 × g for 5 minutes, and suspended in 1 mℓ of TSMC buffer solution. The suspension was centrifugally washed and the washed cells were resuspended in 0.1 mℓ of TSMC buffer solution. To the cell suspension was added 100 μℓ of a 1:1 mixture of TSMC buffer solution at a 2-fold concentration and the said ligase reaction solution, and then admixed with 0.8 mℓ of TSMC buffer solution containing 20% PEG6,000. After three minutes, 2 mℓ of RCGP medium (pH 7.2) was added to the mixture, and the mixture was centrifuged at 2,500 × g for 5 minutes to remove a supernatant. Then, the precipitated protoplast was suspended in 1 mℓ of RCGP medium, and 0.2 mℓ of the cell suspension was spread onto RCGP agar medium containing 200 μg/mℓ kanamycin and cultured at 30°C for 7 days.

Colonies grown on the agar medium were collected by scraping, centrifugally washed twice with physiological saline solution, and suspended in 1 mℓ of physiological saline solution. The cell suspension was respread onto a minimal agar medium M1 containing 800 μg/mℓ PFP and 10 μg/mℓ kanamycin and cultured at 30°C for 5 days. Transformants having PFP-and kanamycin-resistance were selected.

The transformants were cultured in NB medium, and plasmid DNA was isolated from the collected cultured cells in the same manner as in isolation of pCE53 in Example 2, step (1). A plasmid obtained from one of the transformants and named pCDS1 was found to have a structure wherein a SaII-cleaved DNA fragment of 6.7 kb is inserted in pCE53 at the SaII-cleavage site by analysis of digested products with various restriction enzymes by agarose gel electrophoresis (see Fig. 2).

DS activity of Corynebacterium glutamicum ATCC 39019 and the transformant carrying pCDS1 was determined in the same manner as in Example 1, step (5).

DS activity of the transformant carrying pCDS1 was about 10-fold higher than that of ATCC 39019 strain, and was released from inhibition by phenylalanine and tyrosine. Thus, it was found that DS gene derived from Corynebacterium glutamicum K38 strain was encoded on the DNA fragment of 6.7 kb inserted in pCDS1.

(3) Subcloning of DNA fragment containing CM gene:

At first, 5 units of SaII (product of Takara Shuzo Co., Ltd.) was added to 100 μℓ of a reaction solution for restriction enzyme Sal I containing 3 μg of pCS-CM2 plasmid DNA prepared in Example 2, step (1), and the mixture was subjected to reaction at 37°C for 60 minutes. The reaction was stopped by heating the mixture at 65°C for 10 minutes. Separately, 5 units of SaII was added to 100 μℓ of a reaction solution for restriction enzyme SaII containing 3 μg of pCE53 plasmid DNA, and the mixture was subjected to reaction at 37°C for 60 minutes. The reaction was stopped by heating the mixture at 65°C for 10 minutes.

The two reaction mixtures were mixed, and 40 μℓ of a buffer solution for T4 ligase at a 10-fold concentration, 40 μℓ of 5 mM ATP, 0.3 μℓ of T4 ligase (1 unit/μℓ, product of Takara Shuzo Co., Ltd.) and 120 μℓ of water were added thereto. Then, the mixture was subjected to reaction at 12°C for 16 hours.

The thus obtained ligase reaction mixture was used to transformation of Corynebacterium glutamicum KY9457 (having phenylalanine-and tyrosine-requirement due to deficiency of CM activity) [Agric. Biol. Chem., 39, 331 (1975)] (deposited with the FRI on August 7, 1986 as FERM BP-1123).

Protoplast prepared in the following manner was used for the transformation. At first, 4 mℓ of a seed culture of KY9457 strain was inoculated in 40 mℓ of SSM medium containing 100 μg/mℓ phenylalanine and tyrosine, and cultured by shaking at 30°C. When OD reached 0.2, penicillin G was added thereto to a concentration of 0.5 unit/mℓ, and the culturing was continued. When OD reached 0.6, the cells were collected and treated with lysozyme in the same manner as in Example 2, step (2) to prepare protoplasts. The protoplasts were transformed with the said ligase reaction mixture in the same manner as in Example 2, step (2). Kanamycin-resistant colonies grown on RCGP agar medium containing 200 μg/mℓ kanamycin were collected by scraping, centrifugally washed twice with physiological saline solution and suspended in 1 mℓ of physiological saline solution. The thus obtained cell suspension was respread onto minimal agar medium M1 containing 10 μg/mℓ kanamycin and cultured at 30°C for 3 days. Kanamycin-resistant and phenylalanine-and tyrosin-non-requiring transformants were selected. The transformants were cultured in SSM medium and treated with penicillin G in the same manner as above, and plasmid DNA was isolated from the collected, cultured cells in the same manner as in isolation of pCE53 in Example 2, step (1). Plasmid pCCM1 obtained from one of the transformants was digested with various restriction enzymes and analyzed by agarose gel electrophoresis. The plasmid was found to have a structure wherein a SaII-cleaved DNA fragment of 1.9 kb was inserted in pCE53 at the SaII-cleavage site (see Fig. 2).

(4) Cloning of DNA fragment containing PD gene:

At first, 6 units of BamHI (product of Takara Shuzo Co., Ltd.) was added to 60 μℓ of a reaction solution for restriction enzyme Bam HI (comprising 10 mM Tris, 6 mM MgCl₂ and 100 mM NaCl, pH 7.5) containing 3 μg of pCE53 plasmid DNA prepared in Example 2, step (1), and the mixture was subjected to reaction at 37°C for 60 minutes. The reaction was stopped by heating the mixture at 65°C for 10 minutes. Separately, 6 units of BamHI was added to 140 μℓ of a reaction solution for BamHI containing 3 μg of chromosomal DNA of Corynebacterium glutamicum K38 strain, and the mixture was subjected to reaction at 37°C for 60 minutes. The reaction was stopped by heating the mixture at 65°C for 10 minutes. The two reaction mixtures were mixed, and 40 µℓ of T4 ligase reaction buffer solution at a 10-fold concentration, 40 µℓ of 5 mM ATP, 0.3 µℓ of T4 ligase (1 unit/µℓ, product of Takara Shuzo, Co., Ltd.) and 120 µℓ of water were added to the mixture. Then, the mixture was subjected to reaction at 12°C for 16 hours.

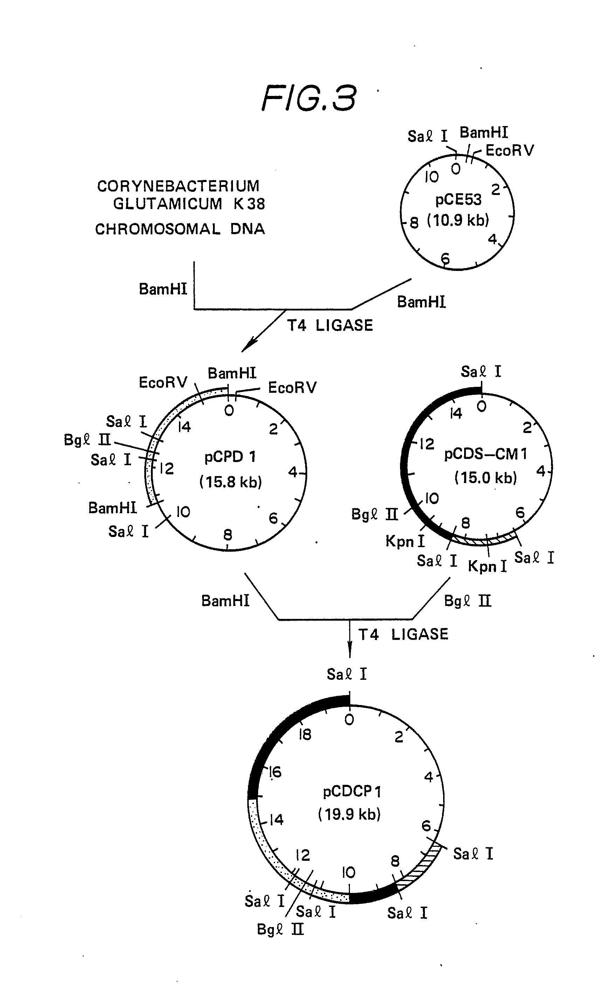

Corynebacterium glutamicumKY9182 (having a phenylalanine-requirement due to deficiency of PD activity) [Agric. Biol. Chem., 39, 331 (1975)] was transformed with the thus obtained ligase reaction mixture in the same manner as in Example 2, step (3). Kanamycin-resistant colonies grown on RCGP agar medium containing 200 µg/mℓ kanamycin were collected by scraping, centrifugally washed twice with physiological saline solution, and suspended in 1 mℓ of physiological saline solution. The cell suspension was respread onto a minimal agar medium M1 containing 10 µg/mℓ kanamycin, and cultured at 30°C for 3 days. Kanamycin-resistant and phenylalanine-non-requiring transformants were selected. The transformants were cultured in SSM medium and treated with penicillin G. Plasmid DNA was isolated from the transformant in the same manner as in isolation of pCE53 in Example 2, step (1). Plasmid pCPD1 obtained from one of the transformants was digested with various restriction enzymes and analyzed by agarose gel electrophoresis. The plasmid was found to have a structure wherein a BamHI-cleaved DNA fragment of 4.9 kb was inserted in pCE53 at the BamHI-cleavage site (see Fig. 3).

(5) Construction of recombinant plasmid containing both DS and CM genes:

A DNA fragment containing DS gene is isolated from pCDS1 and a DNA fragment containing CM gene is isolated from pCCM1, and both DNA fragments are inserted into pCG115.

At first, 5 units of SalI (product of Takara Shuzo Co., Ltd.) was added to 100 µℓ of reaction solution for SalI containing 3 µg of pCDS1, pCCM1 or pCG115 plasmid DNA, respectively, and each of the mixtures was subjected to reaction at 37°C for 60 minutes. The reaction was stopped by heating the mixtures at 65°C for 10 minutes.

The three reaction solutions were mixed, and 40 µℓ of a buffer solution for T4 ligase at a 10-fold concentration, 40 µℓ of 5 mM ATP, 1 µℓ of T4 ligase (1 unit/µℓ, product of Takara Shuzo Co., Ltd.) and 20 µℓ of water were added thereto. The mixture was subjected to reaction at 12°C for 16 hours.

Corynebacterium glutamicum KY9457 (FERM BP-1123) deficient in CM activity was transformed with the thus obtained ligase reaction mixture in the same manner as in Example 2, step (3). Spectinomycin-resistant colonies grown on RCGP agar medium containing 400 µg/mℓ spectinomycin were collected by scraping, centrifugally washed twice with physiological saline solution, and suspended in 1 mℓ of physiological saline solution. The cell suspension was respread onto minimal agar medium MI containing 3 mg/mℓ PFP and 100 µg/mℓ spectinomycin and cultured at 30°C for 5 days. Spectinomycin-and PFP-resistant, and phenylalanine-and tyrosine-non-requiring transformants were selected. Plasmid DNAs were isolated from the transformants in the same manner as in Example 2, step (3). Plasmid pCDS-CM1 obtained from one of the transformants was digested with various restriction enzymes and analyzed by agarose gel electrophoresis. The plasmid was found to have a structure wherein a SalI-cleaved DNA fragment of 6.7 kb containing DS gene and a SalI-cleaved DNA fragment of 1.9 kb containing CM gene were inserted in pCG115 at the SalI-cleavage site (see Fig. 2). The transformant that carries pCDS-CM1 was deposited with the FRI as Corynebacterium glutamicum K-68 (FERM BP-1122) on August 7, 1986.

(6) Construction of recombinant plasmid containing all of DS, CM and PD genes:

A DNA fragment containing PD gene is isolated from pCPD1 and is inserted into pCDS-CM1.

At first, 5 units of BamHI (product of Takara Shuzo Co., Ltd.) was added to 100 µℓ of a reaction solution for BamHI containing 3 µg of pCPD1 plasmid DNA and the mixture was subjected to reaction at 37°C for 60 minutes. The reaction was stopped by heating the mixture at 65°C for 10 minutes. Separately, 5 units of BglII (product of Takara Shuzo Co., Ltd.) was added to 100 µℓ of a reaction solution for BglII (comprising 10 mM Tris, 6 mM MgCl₂ and 100 mM NaCl, pH 7.5) containing 3 µg of pCDS-CM1 plasmid DNA, and the mixture was subjected to reaction at 37°C for 60 minutes. The reaction was stopped by phenol-treatment. The two reaction solutions were mixed, and a double volume of ethanol was added to the mixture. The mixture was left standing at -20°C for 6 hours, and the precipitated DNA was recovered by centrifugation and dissolved in 100 µℓ of TES buffer solution.

Then, 20 μℓ of a buffer solution for T4 ligase at a 10-fold concentration, 20 μℓ of 5 mM ATP, 0.3 μℓ of T4 ligase (1 unit/μℓ, product of Takara Shuzo Co., Ltd.) and 60 μℓ of water were added to the reaction solution, and the mixture was subjected to reaction at 12°C for 16 hours.

Corynebacterium glutamicum KY9182 strain deficient in PD activity was transformed with the thus obtained ligase reaction mixture in the same manner as in Example 2, step (3). Spectinomycin-resistant colonies grown on RCGP agar medium containing 400 μg/ml spectinomycin were collected by scraping, centrifugally washed twice with physiological saline solution and suspended in 1 ml of physiological saline solution. The cell suspension was respread on minimal agar medium MI containing 100 μg/ml spectinomycin, and cultured at 30°C for 3 days. Spectinomycin-resistant, and phenylalanine-non-requiring transformants were selected. Plasmid DNAs were isolated from these transformants in the same manner as in Example 2, step (3). Plasmid pCDCP1 obtained from one of the transformants was digested with various restriction enzymes and analyzed by agarose gel electrophoresis. The plasmid was found to have a structure wherein a BamHI-cleaved DNA fragment of 4.9 kb containing PD gene was inserted in pCDS-CM1 at the BglII-cleavage site (see Fig. 3).

DS, CM and PD activities of Corynebacterium glutamicum ATCC 39019 and the transformant containing pCDCP1 were determined in the same manner as in Example 2, step (5). Activities of DS, CM and PD of the transformant carrying pCDCP1 shown in Table 4 are expressed relative to that of Corynebacterium glutamicum ATCC 39019.

Table 4

| Strains | DS | CM | PD |
|---|---|---|---|
| Corynebacterium glutamicum ATCC 39019 | 1.0 | 1.0 | 1.0 |
| Corynebacterium glutamicum ATCC 39019/pCDCP1 | 9.8 | 11.3 | 11.1 |

(7) Production of phenylalanine by strains carrying pCDCP1:

Phenylalanine-producing strain Corynebacterium glutamicum K38 (FERM BP-454), tryptophan-producing strain Corynebacterium glutamicum K-55 (FERM BP-864) and tyrosine-producing strain Corynebacterium glutamicum K43 (FERM BP-457) were transformed with pCDCP1 in the same manner as in Example 2, step (3) to obtain spectinomycin-resistant transformants. Plasmid DNAs were isolated from the transformants in the same manner as in Example 2, step (3) and analyzed by digestion with various restriction enzymes. The plasmids were found to be the same plasmids as pCDCP1.

Phenylalanine, tyrosine and tryptophane production tests of the transformants and the respective parent strains were carried out in the same manner as in Example 1, step (6), and yields of L-phenylalanine, L-tyrosine and L-tryptophan were determined by paper chromatography.

Results are shown in Table 5.

Table 5

| Strains | L-phenylalanine (g/ℓ) | L-tyrosine (g/ℓ) | L-tryptophan (g/ℓ) |
|---|---|---|---|
| Corynebacterium glutamicum K38 | 5.7 | 0 | 0 |
| Corynebacterium glutamicum K38/pCDCP1 | 10.1 | 0 | 0 |
| Corynebacterium glutamicum K-55 | 0 | 0 | 4.7 |
| Corynebacterium glutamicum K-55/pCDCP1 | 7.2 | 0 | 0.6 |
| Corynebacterium glutamicum K43 | 0 | 5.2 | 0 |
| Corynebacterium glutamicum K43/pCDCP1 | 7.4 | 0 | 0 |

Reference Example

(1) Preparation of plasmid DNA containing pheA gene coding for CM and PD of Escherichia coli:

As disclosed in JP-A-34197/85 and EP-A-136359, a DNA fragment containing wild-type pheA gene originating from Escherichia coli was cloned as an EcoRI-HindIII-cleaved DNA fragment of 4.2 kb, using chromosomal DNA of Escherichia coli JA194 strain derived from Escherichia coli K-12 strain [Proc. Natl. Acad. Sci., 94, 487 (1977)] as a source and pBR322 as a vector. The recombinant plasmid pEaroF1 containing the DNA fragment thus obtained has cleavage sites for various restriction enzymes such as EcoRI, BamHI, HindIII, etc. and it was found by comparison with the DNA fragment cloned by Zlowsky, et al. [Proc. Natl. Acad. Sci., USA, 75, 4271 (1978)] that not only pheA gene but also aroF and tyrA genes were located on the cloned DNA fragment. Thus, in order to subclone a DNA fragment containing only pheA gene from pEaroF1, pEaroF1 was at first isolated from the cultured cells of Escherichia coli AB3248 strain carrying pEaroF1 (a process for preparing the strain is disclosed in JP-A-34197/85 and EP-A-136359) in the same manner as in Example 1, step (1).

(2) Subcloning of pheA gene:

A DNA fragment containing pheA gene is isolated from plasmid pEaroF1 DNA prepared as above and is inserted into vector pBR322 autonomously replicable in Escherichia coli.

At first, 5 units each of restriction enzymes EcoRI and BamHI (products of Takara Shuzo Co., Ltd.) were added to 100 μℓ of a reaction solution for restriction enzymes EcoRI and BamHI (comprising 10 mM Tris, 6 mM MgCl₂ and 100 mM NaCl, pH 7.5) containing 3 μg of plasmid pEaroF1 DNA, and the mixture was subjected to reaction at 37°C for 60 minutes. Separately, 5 units each of EcoRI and BamHI were added to 100 μℓ of a reaction solution for restriction enzymes EcoRI and Bam HI containing 3 μg of pBR322 DNA, and the mixture was subjected to reaction at 37°C for 60 minutes. The reaction was stopped by heating the respective mixtures at 65°C for 10 minutes. The two reaction solutions were mixed, and 40 μℓ of a buffer

15

solution for T4 ligase (comprising 660 mM Tris, 66 mM MgCl₂ and 100 mM dithiothreitol, pH 7.6), 40 $\mu\ell$ of 5 mM ATP, 0.4 $\mu\ell$ of T4 ligase (1 unit/$\mu\ell$, product of Takara Shuzo Co., Ltd.) and 120 $\mu\ell$ of water were added to the mixture. The mixture was subjected to reaction at 12°C for 16 hours. The reaction was stopped by heating the mixture at 65°C for 15 minutes. Escherichia coli GS245 strain derived from Escherichia coli K12 strain (pheA905, araD139,) ΔlacU169, ΔglyA, strA, thi) [Gene, 14, 63, (1981)] was transformed with the thus obtained ligase reaction mixture in the same manner as in Example 1, step (2).

The cells were centrifugally washed twice with physiological saline solution, and spread onto M9 plate medium containing 50 μg/mℓ glycine. Colonies grown on M9 plate medium were spread onto LB plate medium containing 100 μg/mℓ ampicillin and onto LB plate medium containing 20 μg/mℓ tetracycline, and transformants grown on the ampicillin-containing medium and failing to grow on the tetracycline-containing medium were selected.

Plasmid DNA was isolated from the thus selected ampicillin-resistant, and tetracycline-sensitive transformants grown on M9 plate medium containing glycine in the same manner as in Example 1, step (1). Plasmid pPHEA1 obtained from one of the transformants was digested with various restriction enzymes and analyzed by agarose gel electrophoresis. The plasmid was found to have a structure wherein EcoRI-BamHI-cleaved DNA fragment of 1.8 kb is inserted in a region between Eco RI-and BamHI-cleavage sites of pBR322.

## Claims

1. A process for producing L-phenylalanine which comprises introducing, into a microorganism belonging to the genus Corynebacterium or Brevibacterium, a recombinant DNA containing a DNA fragment bearing all of genetic information responsible for synthesis of 3-deoxy-D-arabino-hepturosonate-7-phosphate synthase, chorismate mutase and prephenate dehydratase; culturing the transformant thus obtained in a culture medium until a recoverable amount of L-phenylalanine is accumulated in the culture broth, and recovering L-phenylalanine therefrom.

2. A process according to Claim 1, wherein the microorganism has an ability to produce L-tryptophan or L-tyrosine.

3. A process according to Claim 1, wherein the DNA fragment is isolated from a microorganism belonging to the genus Escherichia, Corynebacterium or Brevibacterium .

4. A recombinant DNA containing a DNA fragment which is isolated from a microorganism belonging to the genus Corynebacterium , Brevibacterium or Escherichia, and bears all of genetic information responsible for synthesis of 3-deoxy-D-arabino-hepturosonate-7-phosphate synthase, chorismate mutase and prephenate dehydratase and being capable of conferring resistance to phenylalanine analogue on a microorganism belonging to the genus Corynebacterium or Brevibacterium.

5. A microorganism belonging to the genus Corynebacterium or Brevibacterium carrying a recombinant DNA containing a DNA fragment which is isolated from a microorganism belonging to the genus Corynebacterium, Brevibacterium or Escherichia ,and bears all of genetic information responsible for synthesis of 3-deoxy-D-arabino-hepturosonate-7-phosphate synthase, chorismate mutase and prephenate dehydratase.

6. Corynebacterium glutamicum K66 (FERM BP-1120).

7. Corynebacterium glutamicum K67 (FERM BP-1121).

8. Corynebacterium glutamicum K68 (FERM BP-1122).

9. Corynebacterium glutamicum KY9457 (FERM BP-1123).

# FIG.1

# FIG.2

FIG.3

CORYNEBACTERIUM
GLUTAMICUM K38
CHROMOSOMAL DNA

Sal I  BamHI
EcoRV
pCE53
(10.9 kb)

BamHI          BamHI
T4 LIGASE

pCPD 1
(15.8 kb)

pCDS—CM1
(15.0 kb)

BamHI          BglII
T4 LIGASE

pCDCP1
(19.9 kb)